# EUROPÄISCHE PATENTANMELDUNG

(11) **EP 2 644 704 A1**
(43) Veröffentlichungstag der Anmeldung: **02.10.2013**
(21) Anmeldenummer: 12161628.8
(22) Anmeldetag: 27.03.2012
(51) Int. Cl.: C12Q 1/68, G01N 33/68

(54) **Markersequenzen für Rheumatoide Arthritis**

(71) Anmelder: Protagen AG, 44227 Dortmund (DE)
(72) Erfinder: Lüking, Angelika, 44892 Bochum (DE)
(74) Vertreter: Simandi, Claus

(57) **Zusammenfassung**

Die vorliegende Erfindung betrifft neue Markersequenzen für Rheumatoide Arthritis und deren diagnostische Verwendung samt einem Verfahren zum Screenen von potentiellen Wirkstoffen für Rheumatoide Arthritis mittels dieser Markersequenzen. Ferner betrifft die Erfindung eine diagnostische Vorrichtung enthaltend solche Markersequenzen für Rheumatoide Arthritis, insbesondere ein Proteinbiochip und dessen Verwendung.

## Beschreibung

Die vorliegende Erfindung betrifft neue Markersequenzen für Rheumatoide Arthritis und deren diagnostische Verwendung samt einem Verfahren zum Screenen von potentiellen Wirkstoffen für Rheumatoide Arthritis mittels dieser Markersequenzen. Ferner betrifft die Erfindung eine diagnostische Vorrichtung enthaltend solche Markersequenzen für Rheumatoide Arthritis, insbesondere einen Proteinbiochip oder Beads (Kügelchen) und deren Verwendung.

Proteinbiochips gewinnen eine zunehmende industrielle Bedeutung in der Analytik und Diagnostik sowie in der Pharmaentwicklung. Proteinbiochips haben sich als Screeninginstrumente etabliert.

Hierbei wird die schnelle und hochparallele Detektion einer Vielzahl spezifisch bindender Analysemoleküle in einem einzigen Experiment ermöglicht. Zur Herstellung von Proteinbiochips ist es erforderlich, die benötigten Proteine zur Verfügung zu haben. Hierzu haben sich insbesondere Protein-Expressionsbibliotheken etabliert. Die Hochdurchsatz-Klonierung von definierten offenen Leserahmen ist eine Möglichkeit (Heyman, J.A., Cornthwaite, J., Foncerrada, L., Gilmore, J.R., Gontang, E., Hartman, K.J., Hernandez, C.L., Hood, R., Hull, H.M., Lee, W.Y., Marcil, R., Marsh, E.J., Mudd, K.M., Patino, M.J., Purcell, T.J., Rowland, J.J., Sindici, M.L. and Hoeffler, J.P. (1999) Genome-scale cloning and expression of individual open reading frames using topoisomerase I-mediated ligation. Genome Res, 9, 383-392; Kersten, B., Feilner, T., Kramer, A., Wehrmeyer, S., Possling, A., Witt, I., Zanor, M.I., Stracke, R., Lueking, A., Kreutzberger, J., Lehrach, H. and Cahill, D.J. (2003) Generation of Arabidopsis protein chip for antibody and serum screening. Plant Molecular Biology, 52, 999-1010; Reboul, J., Vaglio, P., Rual, J.F., Lamesch, P., Martinez, M., Armstrong, C.M., Li, S., Jacotot, L., Bertin, N., Janky, R., Moore, T., Hudson, J.R., Jr., Hartley, J.L., Brasch, M.A., Vandenhaute, J., Boulton, S., Endress, G.A., Jenna, S., Chevet, E., Papasotiropoulos, V., Tolias, P.P., Ptacek, J., Snyder, M., Huang, R., Chance, M.R., Lee, H., Doucette-Stamm, L., Hill, D.E. and Vidal, M. (2003) C. elegans ORFeome version 1.1: experimental verification of the genome annotation and resource for proteome-scale protein expression. Nat Genet, 34, 35-41.; Walhout, A.J., Temple, G.F., Brasch, M.A., Hartley, J.L., Lorson, M.A., van den Heuvel, S. and Vidal, M. (2000) GATEWAY recombinational cloning: application to the cloning of large numbers of open reading frames or ORFeomes. Methods Enzymol, 328, 575-592). Allerdings hängt ein solcher Ansatz stark mit dem Fortschritt der Genom-Sequenzierungsprojekte und der Annotierung dieser Gensequenzen zusammen. Darüber hinaus ist die Bestimmung der exprimierten Sequenz aufgrund differenzieller Spleißvorgänge nicht immer eindeutig. Dieses Problem kann durch die Anwendung von cDNA-Expressionsbibliotheken umgangen werden (Büssow, K., Cahill, D., Nietfeld, W., Bancroft, D., Scherzinger, E., Lehrach, H. and Walter, G. (1998) A method for global protein expression and antibody screening on high-density filters of an arrayed cDNA library. Nucleic Acids Research, 26, 5007-5008; Büssow, K., Nordhoff, E., Lübbert, C., Lehrach, H. and Walter, G. (2000) A human cDNA library for high-throughput protein expression screening. Genomics, 65, 1-8; Holz, C., Lueking, A., Bovekamp, L., Gutjahr, C., Bolotina, N., Lehrach, H. and Cahill, D.J. (2001) A human cDNA expression library in yeast enriched for open reading frames. Genome Res, 11, 1730-1735; Lueking, A., Holz, C., Gotthold, C., Lehrach, H. and Cahill, D. (2000) A system for dual protein expression in Pichia pastoris and Escherichia coli, Protein Expr. Purif., 20, 372-378). Hierbei wird die cDNA eines bestimmten Gewebes in einen bakteriellen oder einen eukaryotischen Expressionsvektor, wie z.B. Hefe, einkloniert. Die für die Expression verwendeten Vektoren zeichnen sich im Allgemeinen dadurch aus, dass sie induzierbare Promotoren tragen, mit denen sich der Zeitpunkt der Proteinexpression steuern lässt. Darüber hinaus weisen Expressionsvektoren Sequenzen für so genannte Affinitätsepitope oder -proteine auf, die zum einen den spezifischen Nachweis der rekombinanten Fusions-Proteine mittels eines gegen das Affinitätsepitop gerichteten Antikörpers erlauben, zum anderen wird die spezifische Aufreinigung über Affinitätschromatographie (IMAC) ermöglicht.

Beispielsweise wurden die Genprodukte einer cDNA-Expressionsbibliothek aus humanem fötalem Hirngewebe in dem bakteriellen Expressionssystem Escherichia coli im Hochdichte-Format auf einer Membran angeordnet und konnten erfolgreich mit unterschiedlichen Antikörpern gescreent werden. Es konnte gezeigt werden, dass der Anteil an Volllänge-Proteinen bei mindestens 66% liegt. Die rekombinanten Proteine aus Expressionsbibliothek konnten darüber hinaus im Hochdurchsatz exprimiert und aufgereinigt werden (Braun P., Hu, Y., Shen, B., Halleck, A., Koundinya, M., Harlow, E. and LaBaer, J. (2002) Proteome-scale purification of human proteins from bacteria. Proc Natl Acad Sci U S A, 99, 2654-2659; Büssow (2000) supra; Lueking, A., Horn, M., Eickhoff, H., Büssow, K., Lehrach, H. and Walter, G. (1999) Protein microarrays for gene expression and antibody screening. Analytical Biochemistry, 270, 103-111). Solche Proteinbiochips auf der Basis von cDNA-Expressionsbibliotheken sind insbesondere Gegenstand der WO 99/57311 und WO 99/57312.

Ferner sind neben Antigen-präsentierenden Proteinbiochips ebenfalls Antikörper-präsentierende Anordnungen beschrieben (Lal et al (2002) Antibody arrays: An embryonic but rapidly growing technology, DDT, 7, 143-149; Kusnezow et al. (2003), Antibody microarrays: An evaluation of production parameters, Proteomics, 3, 254-264).

WO 2009/030226 offenbart bereits Markersequenzen für Rheumatoide Arthritis und deren diagnostische Verwendung samt einem Verfahren zum Screenen von potentiellen Wirkstoffen für Rheumatoide Arthritis mittels dieser Markersequenzen. Ferner eine diagnostische Vorrichtung enthaltend solche Markersequenzen für Rheumatoide Arthritis, insbesondere ein Proteinbiochip und dessen Verwendung.

Es besteht jedoch weiterhin ein hohes Bedürfnis indikationsspezifische diagnostische Vorrichtungen für Rheumatoide Arthritis bereitzustellen.

Die Aufgabe der vorliegenden Erfindung ist daher die Bereitstellung von verbesserten Markersequenzen für Rheumatoide Arthritis und deren diagnostische Verwendung.

Die Bereitstellung von spezifischen Markersequenzen erlaubt eine sichere Diagnose und Stratifizierung von Patienten mit Rheumatoider Arthritis, insbesondere mittels eines Proteinbiochips.

Daher betrifft die Erfindung die Verwendung von Verwendung von Markersequenzen zur Diagnose von Rheumatoider Arthritis, wobei mindestens eine Markersequenz ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfasst und / oder ein durch die Sequenzen 1 bis 156 kodiertes Protein oder eine Teilsequenz oder Fragment davon an oder von einem zu untersuchenden Patienten bestimmt wird und wobei die Markersequenz(en) mit einem Verfahren identifiziert wurde(n), umfassend die
a) Identifizierung von Markersequenzkandidaten durch differentielles Screening mit Proteinbiochips aus jeweils einer cDNA-Expressionsbank eines Patienten mit Rheumatoider Arthritis und einem Probanden ohne Rheumatoide Arthritis,
b) Expression der Markersequenzkandidaten zur Herstellung der kodierten Proteine und / oder Teilproteine (Peptide),
c) Herstellung von Luminex-Beads, an die ein oder mehrere der in Schritt c) hergestellten Proteine und / oder Teilproteine (Peptide) gekoppelt sind wobei an die Luminex-Beads gegebenenfalls auch bereits bekannte Biomarker für Rheumatoide Arthritis, beispielsweise CCP, gekoppelt sind,
d) Validierung der Markersequenzkandidaten mittels Proben von Patienten mit Rheumatoider Arthritis und Probanden ohne Rheumatoide Arthritis, und wobei die Validierung gegebenenfalls in Gegenwart der bereits bekannten Biomarker für Rheumatoide Arthritis, beispielsweise CCP, durchgeführt wird.

Eine bevorzugte Ausführungsform der Erfindung betrifft die Verwendung erfindungsgemäßer Markersequenzen zur Diagnose von Rheumatoider Arthritis, wobei mindestens eine Markersequenz ausgewählt aus der Gruppe der Teilsequenzen SEQ ID No. 29 bis 78 und / oder cDNAs SEQ ID No. 108 bis 156 und / oder der genomischen Sequenzen, die eine der Sequenzen SEQ ID No. 29 bis 78 oder 108 bis 156 umfasst und / oder ein durch die Sequenzen 29 bis 78 oder 108 bis 156 kodiertes Protein oder eine Teilsequenz oder Fragment davon an oder von einem zu untersuchenden Patienten bestimmt wird und wobei die Markersequenz(en) mit einem Verfahren identifiziert wurde(n), umfassend die Schritte a) bis d) und wobei die Schritte c) und d) in Gegenwart von CPP durchgeführt wurden und wobei die Markersequenzen sensitiver im Bezug auf die Diagnose von Rheumatoider Arthritis sind als CCP.

Auf diese Wiese kann eine Subgruppe von Patienten identifiziert und überwacht werden, die mit Hilfe des bereits bekannten Markers CCP für Rheumatoide Arthritis nicht identifiziert werden können. Mit Hilfe diese Markersequenzen kann auch Rheumatoide Arthritis in einem früheren Stadium diagnostiziert werden, als mit CCP (Cytochrom c Peroxidase).

Eine weitere Ausführungsform der Erfindung betrifft die Verwendung der erfindungsgemäßen Markersequenzen zur Diagnose von Rheumatoider Arthritis, dadurch gekennzeichnet, dass 2 oder 3, vorzugsweise 4 oder 5, besonders bevorzugt 6, 7 oder 8 oder mehr verschiedene Markersequenzen, beispielsweise 10 bis 20 oder 30 oder mehr verschiedene Markersequenzen an oder von einem zu untersuchenden Patienten bestimmt werden.

Eine weitere Ausführungsform der Erfindung betrifft die Verwendung der erfindungsgemäßen Markersequenz(en)zur Diagnose von Rheumatoider Arthritis, dadurch gekennzeichnet, dass die Bestimmung mittels in-vitro Diagnose erfolgt.

Eine weitere Ausführungsform der Erfindung betrifft die Verwendung der erfindungsgemäßen Markersequenz(en) ausgewählt aus der Gruppe der Teilsequenzen SEQ ID No. 1 bis 78 und /oder der cDNAs SEQ ID No. 79 bis 156 und / oder der genomischen Sequenzen, die eine der Sequenzen 1 bis 156 umfasst und / oder eines durch die Sequenzen 1 bis 156 kodierten Proteins oder einer Teilsequenz oder eines Fragments davon als Diagnostikum, wobei die Markersequenz(en) mit einem Verfahren identifiziert wurde(n), umfassend die Schritte
a) Identifizierung von Markersequenzkandidaten durch differentielles Screening mit Proteinbiochips aus jeweils einer cDNA-Expressionsbank eines Patienten mit Rheumatoider Arthritis und einem Probanden ohne Rheumatoide Arthritis,
b) Expression der Markersequenzkandidaten zur Herstellung der kodierten Proteine und / oder Teilproteine (Peptide),
c) Herstellung von Luminex-Beads, an die ein oder mehrere der in Schritt c) hergestellten Proteine und / oder Teilproteine (Peptide) gekoppelt sind wobei an die Luminex-Beads gegebenenfalls auch bereits bekannte Biomarker für Rheumatoide Arthritis, beispielsweise CCP, gekoppelt sind,
d) Validierung der Markersequenzkandidaten mittels Proben von Patienten mit Rheumatoider Arthritis und Probanden ohne Rheumatoide Arthritis, und wobei die Validierung gegebenenfalls in Gegenwart der bereits bekannten Biomarker für Rheumatoide Arthritis, beispielsweise CCP, durchgeführt wird.

Eine weitere Ausführungsform der Erfindung betrifft die Verwendung der erfindungsgemäßen Markersequenz(en) zur Diagnose von Rheumatoider Arthritis, dadurch gekennzeichnet, dass die Markersequenz(en) auf einem festen Träger aufgebracht wird (werden), wobei der feste Träger beispielsweise ausgewählt wird aus den festen Trägern umfassend Filter, Membranen, Wafer, beispielsweise Silizium-Wafer, Glas, Metall, Kunststoff, Chips, massenspektrometrische Targets, Matrix, Kügelchen (Beads), beispielsweise magnetische, beschichtete oder markierte Kügelchen (Beads), wie Fluorophor-markierte Kügelchen oder Luminex-Beads.

Eine weitere Ausführungsform der Erfindung betrifft Verfahren zur Diagnose von Rheumatoider Arthritis, wobei
a.) mindestens eine Markersequenz ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfasst und / oder eines durch die Sequenzen 1 bis 156 kodierten Proteins oder einer Teilsequenz oder eines Fragments davon auf einem festen Träger, vorzugsweise auf einem Kügelchen (Bead) aufgebracht wird (werden) und
b.) mit Körperflüssigkeit oder Gewebeauszug eines Patienten in Kontakt gebracht wird (werden) und
c.) der Nachweis einer Wechselwirkung der Körperflüssigkeit oder Gewebeauszug mit der (den) Markersequenz(en) aus a.) erfolgt.

Eine weitere Ausführungsform der Erfindung betrifft Verfahren zum Stratifizieren, insbesondere zur Risikostratifizierung, oder zur Therapiesteuerung eines Patienten mit Rheumatoider Arthritis, wobei mindestens eine Markersequenz ausgewählt aus der Gruppe der Teilsequenzen SEQ ID No. 1 bis 78 und / oder der cDNAs SEQ ID No. 79 bis 156 und / oder den genomischen Sequenzen, die eine der Sequenzen 1 bis 156 umfassen und /oder eines durch die Sequenzen 1 bis 156 kodierten Proteins oder einer Teilsequenz oder eines Fragments davon an oder von einem zu untersuchenden Patienten bestimmt wird.

Eine weitere Ausführungsform der Erfindung betrifft erfindungsgemäße Verfahren, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst.

Eine weitere Ausführungsform der Erfindung betrifft Assays und Proteinbiochips bestehend aus einer Anordnung enthaltend mindestens eine Markersequenz ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfassen und / oder eines durch die Sequenzen 1 bis 156 kodierten Proteins oder einer Teilsequenz oder eines Fragments davon.

Eine weitere Ausführungsform der Erfindung betrifft die Verwendung eines erfindungsgemäßen Assays oder Proteinbiochips zum Identifizieren und Charakterisieren einer Substanz für Rheumatoide Arthritis enthaltend Mittel zum Nachweis eines Bindungserfolges, dadurch gekennzeichnet, dass eine Anordnung oder Assay mit a.) mindestens einer zu untersuchenden Substanz in Kontakt gebracht wird und b.) ein Bindungserfolg nachgewiesen wird.

Eine weitere Ausführungsform der Erfindung betrifft Diagnostika zur Diagnose von Rheumatoider Arthritis enthaltend mindestens eine Markersequenz ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfasst und / oder eines durch die Sequenzen 1 bis 156 kodierten Proteins oder einer Teilsequenz oder eines Fragments davon und wobei die Markersequenz(en) mit einem Verfahren identifiziert wurde(n), umfassend die Schritte
a.)Identifizierung von Markersequenzkandidaten durch differentielles Screening mit Proteinbiochips aus jeweils einer cDNA-Expressionsbank eines Patienten mit Rheumatoider Arthritis und einem Probanden ohne Rheumatoide Arthritis,
b.)Expression der Markersequenzkandidaten zur Herstellung der kodierten Proteine und / oder Teilproteine (Peptide),
c.)Herstellung von Luminex-Beads, an die ein oder mehrere der in Schritt c) hergestellten Proteine und /oder Teilproteine (Peptide) gekoppelt sind wobei an die Luminex-Beads gegebenenfalls auch bereits bekannte Biomarker für Rheumatoide Arthritis, beispielsweise CCP, gekoppelt sind,
d.)Validierung der Markersequenzkandidaten mittels Proben von Patienten mit Rheumatoider Arthritis und Probanden ohne Rheumatoide Arthritis, und wobei die Validierung gegebenenfalls in Gegenwart der bereits bekannten Biomarker für Rheumatoide Arthritis, beispielsweise CCP, durchgeführt wird.

Eine weitere Ausführungsform der Erfindung betrifft ein Target zur Behandlung und Therapie von Rheumatoider Arthritis ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfasst und / oder eines durch die Sequenzen 1 bis 156 kodierten Proteins oder einer Teilsequenz oder eines Fragments davon.

Eine weitere Ausführungsform der Erfindung betrifft die Verwendung einer oder mehrerer Markersequenz ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfassen und /oder eines durch die Sequenzen 1 bis 156 kodierten Proteins oder einer Teilsequenz oder eines Fragments davon als Affinitätsmaterial zur Durchführung einer Apherese oder Blutwäsche für Patienten mit Prostatakarzinom.

Eine weitere Ausführungsform der Erfindung betrifft die Verwendung mindestens einer Markersequenz ausgewählt aus der Gruppe der Teilsequenzen SEQ ID No. 29 bis 78 und / oder der cDNAs SEQ ID No. 108 bis 156 und / oder der genomischen Sequenzen, die eine der Sequenzen SEQ ID No. 29 bis 78 oder 108 bis 156 umfassen und / oder eines durch die Sequenzen 29 bis 78 oder 108 bis 156 kodierten Proteins oder einer Teilsequenz oder eines Fragments davon zur Identifizierung einer Subgruppe von Patienten innerhalb der Gruppe der Patienten mit Rheumatoider Arthritis, wobei die Patienten der Subgruppe nicht mittels des Markers CCR identifiziert werden können.

Eine weitere Ausführungsform der Erfindung betrifft einzelne Markersequenzen für Rheumatoide Arthritis, wobei die einzelnen Markersequenzen ausgewählt werden aus der Gruppe umfassend die Teilsequenzen SEQ ID No. 1 bis 78 und die durch die Sequenzen 1 bis 78 kodierten Protein und Teilsequenzen und Fragmente davon.

Die Erfindung betrifft auch die erfindungsgemäße Verwendung der Proteinsequenzen SEQ ID. No. 157-234 sowie Teile dieser Sequenzen.

Die erfindungsgemäßen Markersequenzen konnten mittels differentiellem Screening von Proben von gesunden Probanden mit Patientenproben mit Rheumatoider Arthritis identifiziert werden. Die erfindungsgemäßen Markersequenzen wurden dann exprimiert und nach Kopplung der exprimierten Markersequenzkandidaten an Luminex-Beads mit Hilfe der Luminex-Beads validiert, z.T. vergleichend gegen bekannte Biomarker für Rheumatoide Arthritis und wie in den Ausführungsbeispielen beschrieben. Dadurch konnten hochspezifische Markersequenzen für Rheumatoide Arthritis identifiziert werden.

Der Begriff "Rheumatoide Arthritis (RA)" ist z.B. nach Pschyrembel, de Gruyter, 261. Auflage (2007), Berlin definiert. Erfindungsgemäß umfasst ist ebenfalls die "juvenile idiopathische Arthritis " (ICD-10: M08.-. Abk.: JIA. Ältere Synonyme: Juvenile rheumatoide Arthritis, Juvenile chronische Arthritis, Morbus Still oder volkstümlicher: kindliches Rheuma), die eine Sammelbezeichnung für eine Reihe von vorwiegend gelenkbefallenden Erkrankungen (Arthritis) des rheumatischen Formenkreises im Kindesalter (juvenil) (Definition z.B. nach Pschyrembel, de Gruyter, 261. Auflage (2007), Berlin). Es handelt sich hierbei um eine polygene Erkrankung, die mittels der erfindungsgemäßen Markersequenzen, vorzugsweise von den Markersequenzen SEQ 401-488 besonders vorteilhaft diagnostiziert werden kann.

In einer weiteren Ausführungsform werden mindestens 2 bis 5 oder 10, vorzugsweise 30 bis 50 Markersequenzen oder 50 bis 100 oder mehr Markersequenzen an oder von einem zu untersuchenden Patienten bestimmt.

In einer weiteren Ausführungsform der Erfindung können die erfindungsgemäßen Markersequenzen ebenfalls mit bekannten Biomarker für diese Indikation kombiniert, ergänzt, fusioniert oder erweitert werden.

In einer bevorzugten Ausführungsform erfolgt die Bestimmung der Markersequenzen außerhalb des menschlichen Körpers und die Bestimmung erfolgt in einer ex vivo / in vitro Diagnose.

Ferner betrifft die Erfindung ein Verfahren zur Diagnose von Rheumatoide Arthritis, wobei a.) mindestens eine erfindungsgemäße Markersequenz auf einem festen Träger aufgebracht wird und b.) mit Körperflüssigkeit oder Gewebeauszug eines Patienten in Kontakt gebracht wird und c.) der Nachweis einer Wechselwirkung der Körperflüssigkeit oder Gewebeauszug mit den Markersequenzen aus a.) erfolgt.

Daher betrifft die Erfindung ebenfalls Diagnostika zur Diagnose von Rheumatoide Arthritis jeweils ausgewählt aus der Gruppe SEQ ID No. 1 bis 78 oder jeweils ein dafür kodierendes Protein oder jeweils einer Teilsequenz oder Fragment davon.

Der Nachweis einer solchen Wechselwirkung kann beispielsweise durch eine Sonde, insbesondere durch einen Antikörper erfolgen.

Daher betrifft die Erfindung ebenfalls die Aufgabe eine diagnostische Vorrichtung oder einen Assay, insbesondere einen Proteinbiochip, bereitzustellen, der für die Rheumatoide Arthritis eine Diagnose oder Untersuchung erlaubt.

Ferner betrifft die Erfindung ein Verfahren zum Stratifizieren, insbesondere zur Risikostratifizierung und / oder Therapiesteuerung eines Patienten mit Rheumatoide Arthritis, wobei mindestens eine erfindungsgemäße Markersequenz an einem zu untersuchenden Patienten bestimmt wird.

Ferner umfasst ist die Stratifizierung der Patienten mit Rheumatoide Arthritis in neue oder etablierte Subgruppen der Rheumatoide Arthritis, sowie die sinnvolle Auswahl von Patientengruppen für die klinische Entwicklung von neuen Therapeutika. Der Begriff Therapiesteuerung umfasst ebenfalls die Einteilung von Patienten in Responder und Nicht-Responder bezüglich einer Therapie oder dessen Therapieverlauf.

"Diagnose" im Sinne dieser Erfindung bedeutet die positive Feststellung der Rheumatoide Arthritis mittels der erfindungsgemäßen Markersequenzen sowie die Zuordnung der Patienten zu der Rheumatoide Arthritis. Der Begriff der Diagnose umfasst die medizinische Diagnostik und diesbezügliche Untersuchungen, insbesondere die in-vitro Diagnostik und Labordiagnostik, ebenfalls Proteomics und Nukleinsäureblots. Weitere Untersuchungen können zur Absicherung und zum Ausschluss anderen Krankheiten vonnöten sein. Daher umfasst der Begriff Diagnose ebenfalls die Differentialdiagnose der Rheumatoide Arthritis mittels der erfindungsgemäßen Markersequenzen sowie die Prognose der Rheumatoide Arthritis.

"Stratifizieren (auch: Stratifikation) oder Therapiesteuerung" im Sinne dieser Erfindung bedeutet, dass das erfindungsgemäße Verfahren Entscheidungen zur Behandlung und Therapie des Patienten erlaubt, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten.

In einer weiteren Ausführungsform der Erfindung umfasst der Begriff "Stratifizierung" insbesondere die Risikostratifizierung mit der Prognose eines "outcome" eines nachteiligen gesundheitlichen Ereignisses.

Im Rahmen dieser Erfindung wird unter "Patient" ein beliebiger Proband - Mensch oder Säugetier - verstanden, mit der Maßgabe, dass der Proband auf Rheumatoide Arthritis untersucht wird.

Der Begriff "Markersequenzen" im Sinne dieser Erfindung bedeutet, dass die cDNA oder das jeweils daraus erhältliche Polypeptid oder Protein signifikant für Rheumatoide Arthritis sind. Beispielsweise können die cDNA oder das jeweils daraus erhältliche Polypeptid oder Protein eine Wechselwirkung mit Substanzen aus der Körperflüssigkeit oder Gewebeauszug eines Patienten mit Rheumatoide Arthritis aufweisen (z.B. Antigen (Epitop) / Antikörper (Paratop) Wechselwirkung). Im Sinne der Erfindung bedeutet "wobei mindestens eine Markersequenz einer ausgewählt aus der Gruppe der Teilsequenzen SEQ ID No. 1 bis 78, der cDNAs SEQ ID No. 79 bis 156 oder ein dadurch kodiertes Protein oder eine Teilsequenz oder Fragment davon an einem zu untersuchenden Patienten bestimmt wird", dass eine Wechselwirkung zwischen der Körperflüssigkeit oder Gewebeauszuges eines Patienten und den erfindungsgemäßen Markersequenzen nachgewiesen wird. Eine solche Wechselwirkung ist z.B. eine Bindung, insbesondere eine bindende Substanz an mindestens einer erfindungsgemäßen Markersequenz oder im Fall einer cDNA die Hybridisierung mit einer geeigneten Substanz unter gewählten Bedingungen, insbesondere stringenten Bedingungen (z.B. wie üblich definiert in J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur.

Solche Substanzen sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten. In einer weiteren Ausführungsform der Erfindung können jedoch die erfindungsgemäßen Markersequenzen in einer signifikant höheren oder niedrigeren Expressionsrate oder Konzentration vorliegen, dass auf die Rheumatoide Arthritis hinweist. Hierbei wird mittels Proteomics oder Nukleinsäureblots die relativen Expressionsraten krank / gesund der erfindungsgemäßen Markersequenzen für Rheumatoide Arthritis bestimmt.

Die Markersequenzen verfügen in einer weiteren Ausführungsform der Erfindung über ein Erkennungssignal, welches an die zu bindende Substanz adressiert ist (z.B. Antikörper, Nukleinsäure). Erfindungsgemäß bevorzugt ist für ein Protein das Erkennungssignal ein Epitop und / oder Paratop und / oder Hapten und für eine cDNA eine Hybridisierungs- oder Bindungsregion.

Die erfindungsgemäßen Markersequenzen sind Gegenstand der Tabelle A und können durch den jeweilig zitierten Datenbankeintrag (auch mittels Internet: http://www.ncbi.nlm.nih.gov/) eindeutig identifiziert werden (siehe in den Tabellen: dort Accession No.).

Daher betrifft die Erfindung ebenfalls die Volllängesequenzen der erfindungsgemäßen Markersequenzen und die Markersequenzen wie in den Tabellen über die bekannten Datenbankeinträge definiert sowie die im anliegenden Sequenzprotokoll angegebenen.

Weiterhin umfasst sind daher ebenfalls analoge Ausführungsformen der Markersequenzen SEQ ID No. 1 bis 234, insbesondere von SEQ ID No. 1 bis 78, wie z.B. in den Ansprüchen dargelegt, da die erfindungsgemäßen SEQ 1-78 wiederum Teilsequenzen, zumindest mit hoher Homologie, darstellen. Weiterhin bevorzugt sind die Sequenzen SEQ ID No. 29 bis 78.

In einer weiteren Ausführungsform der Erfindung sind Markersequenzen bevorzugt, die P-Werte kleiner oder gleich 0.006 aufweisen, vorzugsweise kleiner oder gleich 0.001 oder kleiner oder gleich 0.0001, besonders bevorzugt kleiner oder gleich 0.00001. In einer anderen Ausführungsform der Erfindung sind die Markersequenzen SEQ ID No. SEQ ID No. 5 bis 18 Teilsequenzen, Fragmente oder Homologe sowie die dadurch kodierten Peptide / Proteine bevorzugt, denn diese Markersequenzen weisen besonders geeignete P-Werte auf. Der P-Wert gibt die Wahrscheinlichkeit an, mit der eine Übereinstimmung in der Datenbank gefunden wurde. Für die Definition des P-Werts siehe zum Beispiel http://www.ncbi.nlm.nih.gov/books/NBK62051/.

In einer weiteren Ausführungsform der Erfindung sind ebenfalls Teilsequenzen oder Fragmente der erfindungsgemäßen Markersequenzen umfasst. Insbesondere solche Teilsequenzen, die eine Identität von 99 % oder mehr, 98 %, 97 %, 96 %, 95%, 94 %, 93 %, 92 %, 91 %, 90 %, insbesondere 85 %, 80% oder 70 % mit den erfindungsgemäßen Markersequenzen aufweisen und für die erfindungsgemäße Verwendung - den Nachweis Prostatakarzinom unter Ausschluss von Prostataentzündungserkrankungen oder Diabetes oder Polymorbidität - geeignet sind. (sog. "Homologe", homologe Markersequenzen). Hiomologe können Protein- oder Nukleinsäuresequenzen sein.

Teilsequenzen sind ebenfalls solche Sequenzen, die 50 bis 100 Nukleotide, 70-120 Nukleotide einer Sequenz der SEQ 1-452 aufweisen, oder davon erhältliche Peptide.

Erfindungsgemäß umfassen die Markersequenzen auch solche Modifikationen der cDNA-Sequenz und der entsprechenden Aminosäuresequenz, wie chemische Modifikation, wie Citrullinierung, Acetylierung, Phosphorylierung, Glykosilierung oder polyA-Strang und weiteren dem Fachmann einschlägig bekannte Modifikationen.

In einer weiteren Ausführungsform kann die jeweilige Markersequenz in unterschiedlichen Mengen in einen oder mehreren Bereichen auf einem festen Träger repräsentiert sein. Dies erlaubt eine Variation der Sensitivität. Die Bereiche können jeweils eine Gesamtheit von Markersequenzen aufweisen, d.h. eine genügende Zahl an verschiedenen Markersequenzen, insbesondere 2 bis 5 oder 10 oder mehr und ggfs. weiteren Nukleinsäuren und/oder Proteinen, insbesondere Biomarker. Bevorzugt sind jedoch mindestens 96 bis 25.000 (numerisch) oder mehr aus verschiedenen oder gleichen Markersequenzen und weiteren Nukleinsäuren und/oder Proteinen, insbesondere Biomarker. Weiterhin bevorzugt sind mehr als 2.500, besonders bevorzugt 10.000 oder mehr verschiedene oder gleiche Markersequenzen und ggfs. weiteren Nukleinsäuren und/oder Proteinen, insbesondere Biomarker.

Ein weiterer Gegenstand der Erfindung betrifft eine Anordnung von Markersequenzen enthaltend mindestens eine Markersequenz einer cDNA ausgewählt aus der Gruppe SEQ 1 - 156 oder jeweils ein dadurch kodiertes Protein. Vorzugsweise enthält die Anordnung mindestens 2 bis 5 oder 10, vorzugsweise 30 bis 50 Markersequenzen oder 50 bis 100 oder mehr Markersequenzen.

Im Rahmen dieser Erfindung bedeutet "Anordnung" synonym "Array" und sofern dieser "Array" zur Identifizierung von zu Substanzen an Markersequenzen verwendet wird, ist hierunter ein "Assay" oder diagnostische Vorrichtung zu verstehen. In einer bevorzugten Ausführungsform ist die Anordnung derart gestaltet, dass die auf der Anordnung repräsentierten Markersequenzen in Form eines Gitters auf einem festen Träger vorliegen. Ferner sind solche Anordnungen bevorzugt, die eine hochdichte (high-density) Anordnung von Proteinbindern erlauben und die Markersequenzen gespottet werden. Solche hochdichte gespotteten Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Erfindung umfasst jedoch der Begriff "Assay" oder diagnostische Vorrichtung ebenfalls solche Ausführungsformen einer Vorrichtung, wie ELISA (z.B.einzelne Wells einer Mikrotiterplatte werden mit den erfindungsgemäßen Markersequenzenoder Kombinationen von Markersequenzen beschichtet, ggfs. robotergestützt in die einzelnen Wells der Mikrotiterplatte aufgebracht; Beispiele sind diagnostische ELISA-Kits der Fa. Phadia oder Multiplex-ELISA-Kits "Searchlight" der Fa. Pierce/Thermo Fisher Scientific), Beadbased Assay (spektral unterscheidbare bead-Populationen werden mit Markersequenzen/Kombinationen von Markersequenzen beschichtet. Die Patientenprobe wird mit dieser bead-Population inkubiert und gebundene (Auto-)-Antikörper werden mittels eines weiteren Floureszenzmarkierten Sekundärantikörper/Detektionsreagenz über Messung der Fluoreszenz nachgewiesen; z. B. Borrelia IgG-Kit oder Athena-Multilyte der Fa. Multimetrix) , Line Assay (erfindungsgemäßen Markersequenzen oderKombinationen von Markersequenzen werden robotergestützt auf Membranen immobilisiert, welche mit der Patientenprobe untersucht/inkubiert werden; Beispiel "Euroline" der Fa. Euroimmun AG), Western Blot (Beispiel "Euroline-WB" der Fa. Euroimmun AG), immunchromatographische Verfahren (z.B. Lateral Flow Immunoassays; Markersequenzen/Kombinatione von Markersequenzen werden auf Teststreifen (Membranen, US 5,714,389 u.v.a) immobilisiert; Beispiel "One Step HBsAg" Test Device von Acon Laboratories) oder ähnliche immunologische Single- oder Multiplex-Nachweisverfahren.

Die Markersequenzen der Anordnung sind auf einen festen Träger fixiert, vorzugsweise jedoch gespottet oder immobilisiert gar aufgedruckt, d.h. reproduzierbar aufgebracht. Ein oder mehrere Markersequenzen können mehrfach in der Gesamtheit aller Markersequenzen präsent sein und in unterschiedlichen Mengen bezogen auf einen Spot vorliegen. Ferner können die Markersequenzen auf dem festen Träger standardisiert sein (z.B. mittels serieller Verdünnungsreihen von z.B. Humanglobulinen als interne Kalibratoren zur Datennormalisierung und quantitativen Auswertung).

Daher betrifft die Erfindung einen Assay oder Proteinbiochip bestehend aus einer Anordnung enthaltend erfindungsgemäße Markersequenzen.

In einer weiteren Ausführungsform liegen die Markersequenzen als Clone vor. Solche Clone können beispielsweise mittels einer erfindungsgemäßen cDNA-Expressionsbibliothek erhalten werden (Büssow et al. 1998 (supra)). In einer bevorzugten Ausführungsform werden solche Expressionsbibliotheken enthaltend Clone mittels Expressionsvektoren aus einer exprimierenden cDNA Bibliothek bestehend aus den cDNA Markersequenzen erhalten. Diese Expressionsvektoren enthalten vorzugsweise induzierbare Promotoren. Die Induktion der Expression kann z.B. mittels eines Induktors, solche wie IPTG, erfolgen. Geeignete Expressionsvektoren sind beschrieben in Terpe et al. (Terpe T Appl Microbiol Biotechnol. 2003 Jan; 60(5):523-33).

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe). Ferner sind erfindungsgemäß ebenfalls solche Expressionsbibliotheken mit eingeschlossen, die mittels exontrapping erhalten werden können. Statt Expressionsbibliothek kann synonym von einer Expressionsbank gesprochen werden.

Weiterhin bevorzugt sind Proteinbiochips oder entsprechende Expressionsbibliotheken, die keine Redundanz aufweisen (so genannte: Uniclone®-Bibliothek) und nach den Lehren der WO 99/57311 und WO 99/57312 beispielsweise hergestellt werden können. Diese bevorzugten Uniclone- Bibliotheken weisen einen hohen Anteil an nicht-fehlerhaften vollständig exprimierten Proteinen einer cDNA-Expressionsbibliothek auf.

Im Rahmen dieser Erfindung können die Clone ebenfalls nicht abschließend solche sein, wie transformierte Bakterien, rekombinante Phagen oder transformierte Zellen von Säugern, Insekten, Pilzen, Hefen oder Pflanzen.

Die Clone werden auf einen festen Träger fixiert, gespottet oder immobilisiert.

Daher betrifft die Erfindung eine Anordnung, wobei die Markersequenzen als Clone vorliegen.

Zusätzlich können die Markersequenzen in der jeweiligen Form in Form eines Fusionsproteins vorliegen, welches beispielsweise mindestens ein Affinitätsepitop oder "Tag" enthält. Der Tag kann ein solcher sein wie wie c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA, ein Fusionsprotein, vorzugsweise eine Cellulose-bindende Domäne, grünfluoreszierendes Protein, Maltose bindendes Protein, calmodulin-bindendes Protein, Glutathione S-transferase oder lacZ enthalten.

Eine Markersequenz kann sich auch aus mehreren einzelnen Markersequenzen zusammensetzen. Dies kann die Klonierung einzelner Fragmente zu einem großen gemeinsamen Fragment und die Expression dieses kombinierten Fragmentes beinhalten.

In sämtlichen Ausführungsformen umfasst der Begriff "fester Träger" Ausführungen wie einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix. Ein Filter ist jedoch erfindungsgemäß bevorzugt.

Als Filter ist weiterhin PVDF, Nitrocellulose oder Nylon bevorzugt (z.B. Immobilon P Millipore, Protran Whatman, Hybond N+ Amersham).

In einer weiteren bevorzugten Ausführungsform der erfindungsgemäßen Anordnung entspricht diese einem Gitter, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt.

In einer weiteren bevorzugten Ausführungsform werden Kügelchen sogenannte Beads als Träger verwendet. Vorzugsweise werden dabei bead-basierte multiplex Assays verwendet. Die Analyse und Auswertung der Bead-basierten Assays kann beispielsweise mit einem Luminex-Analysesystem erfolgen, welches auf der Methode der Durchflusszytometrie unter Verwendung zweier unterschiedlicher Laser durchgeführt wird.

Während die Messungen auf planaren Proteinarrays lediglich einen dynamischen Bereich von 1,5 - 2 Größenordnungen (10er Potenzen) bieten, können kann durch die Verwendung von Luminex-beads ein dynamischer Bereich von 3,5-4 Größenordnungen abgedeckt werden. Wobei auch die Messungen in den niedrigen Responsbereichen, sehr gute Variationskoeffizienten (VKs), d.h. nicht mehr als 10% liefern.

Während die Messungen auf planaren Proteinarrays lediglich Variationskoeffizienten (VKs) von 10-25% (intraarray Vergleich) bzw. 10-50% (interarray Vergleich), befinden sich die VKs der Luminexmessungen zwischen 3-10%. Dadurch ergibt sich eine Assayqualität, die kommerzielle ELISAs im Allgemeinen nicht erreichen. Die bekannten Nachteile (limitierte Plexing-Rate durch Interferenz unterschiedlicher Detektionsantikörper) für Luminex-basierte Analyse-und Diagnostikverfahren treffen auf das UNIarray-Konzept nicht zu, da als Detektionssonde lediglich ein einziges fluoreszenzmarkiertes anti-human IgG aus Ziege, Schaf oder Maus eingesetzt wird. Durch den Transfer des UNIarray-Konzeptes auf Luminex (also bead-basierte Protein-Arrays) können zusätzlich mehrere Geräte eingespart, d.h. Protein-Drucker, Hybridisiermaschine und Array-Reader, und durch ein Gerät ersetzt werden. Dabei ist das UNIarray-Konzept nicht an Luminex gebunden, sondern kann auch auf anderen Plattformen wie z.B. Randox, VBC-Genomics etc eingesetzt werden. Die hohe Messgenauigkeit und die niedrigen VKs der Einzelmessungen erlauben nun den Einsatz besserer und neuer statistischer Verfahren zur Identifizierung von potenten Einzelmarkern sowie zur schnellen Aussortierung von Falschpositiven.

In einer weiteren Ausführungsform betrifft die Erfindung einen Assay oder Proteinbiochip zum Identifizieren und Charakterisieren einer Substanz für Rheumatoide Arthritis, dadurch gekennzeichnet, dass eine erfindungsgemäße Anordnung oder Assay mit a.) mindestens einer zu untersuchenden Substanz in Kontakt gebracht wird und b.) ein Bindungserfolg nachgewiesen wird.

Ferner betrifft die Erfindung ein Verfahren zum Identifizieren und Charakterisieren einer Substanz für Rheumatoide Arthritis, dadurch gekennzeichnet, dass eine erfindungsgemäße Anordnung oder Assay mit a.) mindestens einer zu untersuchenden Substanz in Kontakt gebracht wird und b.) ein Bindungserfolg nachgewiesen wird.

Die zu untersuchende Substanz kann ein beliebiges natives oder nicht-natives Biomolekül, ein synthetisches chemisches Molekül, eine Mischung oder eine Substanzbibliothek sein.

Nachdem die zu untersuchende Substanz eine Markersequenz kontaktiert, erfolgt die Auswertung des Bindungserfolges, die beispielsweise unter Verwendung mit handelsüblicher Image-Analyse Software (GenePix Pro (Axon Laboratories), Aida (Raytest), ScanArray (Packard Bioscience) erfolgt.

Die Visualisierung erfindungsgemäßer Protein-Protein-Wechselwirkungen (z.B. Protein an Markersequenz, wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, , kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. mittels eines Microarray-Laserscanners, einer CCD-Kamera oder visuell.

In einer weiteren Ausführungsform betrifft die Erfindung ein Arzneimittel / Wirkstoff oder Prodrug für Rheumatoide Arthritis entwickelt und erhältlich durch den Einsatz des erfindungsgemäßen Assays oder Proteinbiochip.

Daher betrifft die Erfindung ebenfalls die Verwendung einer erfindungsgemäßen Anordnung oder einem Assay zum Screenen von Wirkstoffen für Rheumatoide Arthritis.

Daher betrifft die Erfindung in einer weiteren Ausführungsform ebenfalls ein Target zur Behandlung und Therapie von Rheumatoide Arthritis jeweils ausgewählt aus der Gruppe SEQ 1 - 488 oder jeweils ein dafür kodierendes Protein.

In einer weiteren Ausführungsform betrifft die Erfindung ebenfalls die Verwendung der erfindungsgemäßen Markersequenzen, vorzugsweise in Form einer Anordnung, als Affinitätsmaterial zur Durchführung einer Apherese bzw. iwS. einer Blutwäsche, wobei Substanzen aus Körperflüssigkeiten eines Patienten mit Rheumatoide Arthritis, wie Blut oder Plasma, an die erfindungsgemäßen Markersequenzen binden und folglich der Körperflüssigkeit selektiv entzogen werden können.

Entsprechende Vorrichtungen sind einschlägig bekannt, wie z.B. chromatographische Vorrichtungen enthaltend Beads, Kugeln oder chromatographisches Material, z.B. in einer Säule, die die erfindungsgemäßen Markersequenzen aufweisen und daher z.B. (Auto)Antikörper selektiv entziehen können.

Beispiele und Figuren:
Figur 1: Tabellen 8 und 8a

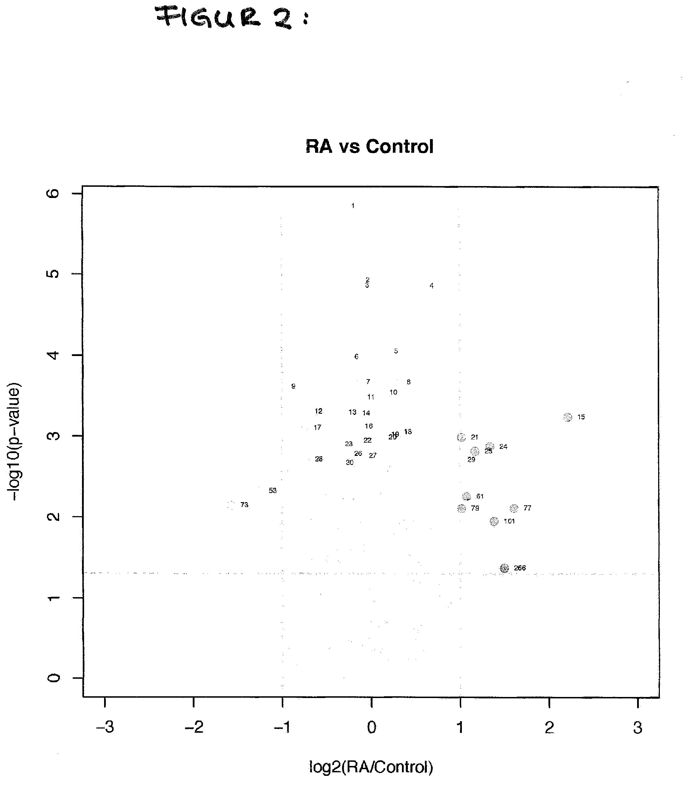

Figur 2: Volcano Plot Rheumatoide Arthritis vs Kontrolle

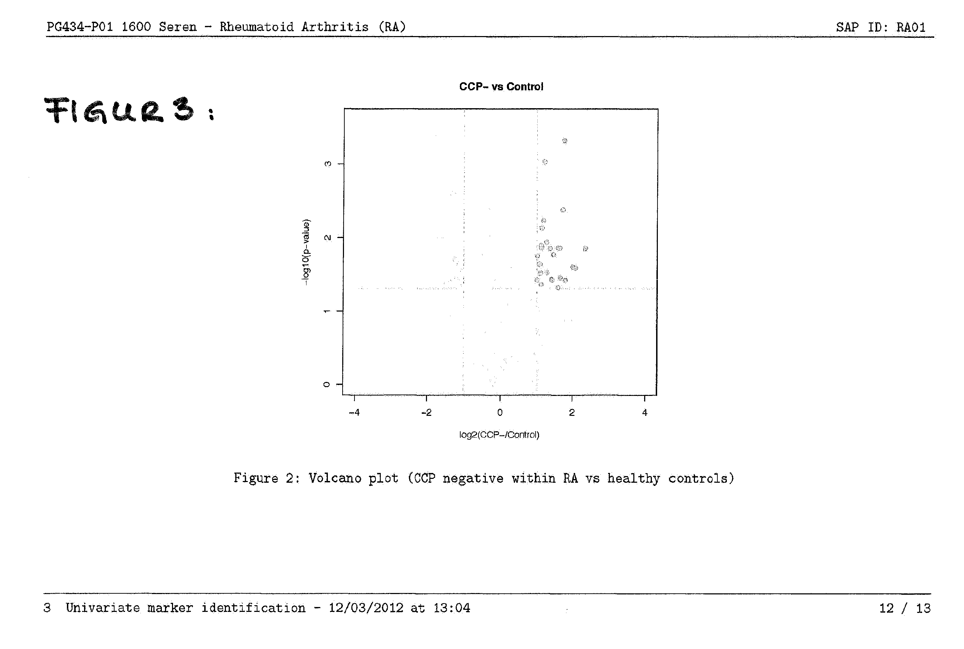

Figur 3: Volcano Plot CCP negativ in der RA Gruppe vs. Kontrolle

### Beispiel 1: Auswahl der Markersequenzkandidaten und Herstellung der Luminex-Beads

Zehn oder mehr Patientenproben wurden individuell gegen eine cDNA Expressionsbibliothek gescreent. Die Rheumatoide Arthritis-spezifischen Expressionsklone wurden ermittelt durch einen Vergleich mit zehn oder mehr gesunden Proben. Die Identität der Markersequenzen wurde durch DNA-Sequenzierung ermittelt.

Es wird ein differentielles Screenen zwischen zwei Proteinbiochips aus jeweils einer cDNA-Expressionsbank eines Patienten und einem gesunden Probanden durchgeführt und die differentiellen Clone werden mittels Fluoresenzmarkierung nachgewiesen und bioinformatorisch ausgewertet.

Es wurden etwa ∼ 3.500 Proteine, die in Untersuchungen mit Proteinbiochips als offenbar wichtige Antigene für entzündlichen Erkrankungen aufgefallen waren, eingesetzt.

Diese 3.500 Proteine wurden daraufhin in größeren Mengen (mehrere mg) hergestellt, gereinigt und auf Luminex-Beads gekoppelt. Zusätzlich wurden bereits bekannte Biomarker (public domain), wie z.B. CCP für Rheumatoide Arthritis, Aquaporin 4 für Neuromyolitis Optica, diverse Zytokine, typische Autoimmunmarker etc. hergestellt und zusammen mit den 3.500 Proteinen gemessen. Die Inklusion von bekannten Autoantigen in Screening und Validierung ist insofern von Bedeutung als hierdurch sehr schnell die besten neuen Kandidaten ausgewählt werden können.

### Beispiel 2: Auswahl der Patienten und Prbanden für die Validierung der Markersequenzkandiaten

Auswahl der Gruppe der zu testenden Personen: Patienten mit Rheumatoider Artritis (RA) und Probanden ohne Rheumatoide Artritis (Control)

**Table 1: Homogenitätsanalyse der Gruppen (Patienten, Probanden)**

| | **Group** | **Attribute** | **p-value** |
|---|---|---|---|
| 1 | RA vs | Age | 0.3996 |
| 2 | Control RA vs Control | Gender | <0.0001 |

**Table 2: Statistische Daten zum Alter der Gruppe**

| | **Group** | **N** | **N miss** | **Mean** | **Median** | **SD** | **Min.** | **Max.** |
|---|---|---|---|---|---|---|---|---|
| 1 | Control | 71 | 0 | 54.62 | 56.00 | 11.32 | 26.00 | 69.00 |
| 2 | RA | 75 | 0 | 56.56 | 57.00 | 13.23 | 25.00 | 89.00 |

**Table 3: Statistische Daten zum Geschlecht der Gruppe**

| | **Group** | **Gender** | **Frequency** | **Percentage** |
|---|---|---|---|---|
| 1 | Control | F | 52 | 73.24 |
| 2 | Control | M | 19 | 26.76 |
| 3 | RA | F | 54 | 72.00 |
| 4 | RA | M | 21 | 28.00 |

**Table 4: Statistische Daten für DAS28**

| | **Group** | **N** | **N miss** | **Mean** | **Median** | **SD** | **Min.** | **Max.** |
|---|---|---|---|---|---|---|---|---|
| 1 | Control | 0 | 71 | | | | | |
| 2 | RA | 55 | 20 | 3,28 | 3.00 | 1.14 | 1.60 | 5.70 |

**Table 5: Statistische Daten für DAS28 im Hinblick auf die Kategorie**

| | **Group** | **DAS 28** | **Range** | **Frequency** | **Percentage** |
|---|---|---|---|---|---|
| 1 | Control | Remission | <2.6 | 0 | 0.00 |
| 2 | Control | Low | 2.6 - 3.1 | 0 | 0.00 |
| 3 | Control | Moderate | >3.2 - 5.1 | 0 | 0.00 |
| 4 | Control | High | >5.1 | 0 | 0.00 |
| 5 | RA | Remission | <2.6 | 17 | 30.91 |
| 6 | RA | Low | 2.6 - 3.1 | 13 | 23.64 |
| 7 | RA | Moderate | >3.2 - 5.1 | 19 | 34.55 |
| 8 | RA | High | >5.1 | 6 | 10.91 |

**Table 6: Statistische Daten im Bezug auf die Dauer der Krankheit**

| | **Group** | **N** | **N miss** | **Mean** | **Median** | **SD** | **Min.** | **Max.** |
|---|---|---|---|---|---|---|---|---|
| 1 | Control | 0 | 71 | | | | | |
| 2 | RA | 71 | 4 | 132.20 | 79.36 | 132.50 | 1.38 | 543.40 |

### Beispiel 3: Identifizierung der erfindungsgemäßen Markersequenzen

Tabelle 7 fasst die Sequenzen SEQ ID No. 1 bis 78 zusammen, die für Rheumatoide Arthritis spezifisch sind und die auch zur Identifizierung der für Rheumatoide Arthritis spezifischen Sequenzen SEQ ID No. 79 bis 156 (siehe Sequenzprotokoll) und die für Rheumatoide Arthritis spezifischen Proteinsequenzen (Teilsequenzen) SEQ ID No. 157 bis 234 (siehe Sequenzprotokoll) verwendet wurden.

**Tabelle 7:**

| SEQ ID No. | Vergleich | GeneID | Gene Name | Gene Symbol | RefSeq Accession | GI Accession |
|---|---|---|---|---|---|---|
| 1 | Control vs RA | 8655 | dynein, light chain, LC8-type 1 | DYNLL1 | ref\|NM_003746 | gi\|4505813 |
| 2 | Control vs RA | 440 | asparagine synthetase (glutamine-hydrolyzing) | ASNS | ref\|NM_133436 | gi\|168229248 |
| 3 | Control vs RA | 79703 | chromosome 11 open reading frame 80 | C11orf80 | ref\|NM_024650 | gi\|170932471 |
| 4 | Control vs RA | 977 | CD151 molecule (Raph blood group) | CD151 | ref\|NM_004357 | gi\|21237748 |
| 5 | Control vs RA | 896 | cyclin D3 | CCND3 | ref\|NM_001760 | gi\|4502619 |
| 6 | Control vs RA | 309 | annexin A6 | ANXA6 | ref\|NM_004033 | gi\|71773415 |
| 7 | Control vs RA | 440354 | PI-3-kinase-related kinase SMG-1 pseudogene | LOC440354 | | gi\|194385888 |
| 8 | Control vs RA | 5909 | RAP1GAP RAP1 GTPase activating protein [Homo sapiens] | RAP1GAP | ref NM_002885 | gi\|4506415 |
| 9 | Control vs RA | 8751 | ADAM metallopeptidase domain 15 | ADAM15 | ref\|NM_207196 | gi\|46909598 |
| 10 | Control vs RA | 1175 | adaptor-related protein complex 2, sigma 1 subunit | AP2S1 | ref\|NM_004069 | gi\|70906430 |
| 11 | Control vs RA | 326625 | methylmalonic aciduria (cobalamin deficiency) cblB type | MMAB | ref\|NM_052845 | gi\|16418349 |
| 12 | Control vs RA | 9129 | PRP3 pre-mRNA processing factor 3 homolog (S. cerevisiae) | PRPF3 | ref\|NM_004698 | gi\|4758556 |
| 13 | Control vs RA | 23170 | tubulin tyrosine ligase-like family, member 12 | TTLL12 | ref\|NM_015140 | gi\|11056036 |
| 14 | Control vs RA | 3098 | hexokinase 1 | HK1 | ref\|NM_033500 | gi\|188497750 |
| 15 | Control vs RA | 81620 | chromatin licensing and DNA replication factor 1 | CDT1 | ref\|NM_030928 | gi\|188497689 |
| 16 | Control vs RA | 8140 | solute carrier family 7 (amino acid transporter light chain, L system), member 5 | SLC7A5 | ref\|NM_003486 | gi\|71979932 |
| 17 | Control vs RA | 27344 | proprotein convertase subtilisin/kexin type 1 inhibitor | PCSK1N | ref\|NM_013271 | gi\|7019519 |
| 18 | Control vs RA | 727910 | TLC domain containing 2 | TLCD2 | | gi\|205830928 |
| 19 | Control vs RA | 5819 | poliovirus receptor-related 2 (herpesvirus entry mediator B) | PVRL2 | ref\|NM_001042724 | gi\|112789532 |
| 20 | Control vs RA | 84196 | ubiquitin specific peptidase 48 | USP48 | ref\|NM_032236 | gi\|52630449 |
| 21 | Control vs RA | 22913 | RNA binding protein, autoantigenic (hnRNP-associated with lethal yellow homolog (mouse)) | RALY | ref\|NM_016732 | gi\|8051631 |
| 22 | Control vs RA | 64221 | roundabout, axon guidance receptor, homolog 3 (Drosophila) | ROBO3 | ref\|NM_022370 | gi\|48476182 |
| 23 | Control vs RA | 728294 | D-2-hydroxyglutarate dehydrogenase | D2HGDH | ref\|NM_152783 | gi\|119964728 |
| 24 | Control vs RA | 79921 | transcription elongation factor A (SII)-like 4 | TCEAL4 | ref\|NM_001006935 | gi\|55749442 |
| 25 | Control vs RA | 147808 | zinc finger protein 784 | ZNF784 | ref\|NM_203374 | gi\|42794622 |
| 26 | Control vs RA | 9040 | UBE2M ubiquitin-conjugating enzyme E2M [ Homo sapiens] | UBE2M | ref\|NM_003969 | gi\|150417997 |
| 27 | Control vs RA | 55778 | ZNF839 zinc finger protein 839 [ Homo sapiens] | ZNF839 | ref\|NM_018335 | gi\|153251839 |
| 28 | Control vs RA | 27344 | proprotein convertase subtilisin/kexin type 1 inhibitor | PCSK1N | ref\|NM_013271 | gi\|7019519 |
| 29 | Control vs RA ccp neg | 10726 | nuclear distribution gene C homolog (A. nidulans) | NUDC | ref\|NM_006600 | gi\|5729953 |
| 30 | Control vs RA ccp neg | 9890 | lipid phosphate phosphatase-related protein type 4 | LPPR4 | ref\|NM_014839 | gi\|33636722 |
| 31 | Control vs RA ccp neg | 1794 | dedicator of cytokinesis 2 | DOCK2 | ref\|NM_004946 | gi\|31377468 |
| 32 | Control vs RA ccp neg | 3931 | lecithin-cholesterol acyltransferase | LCAT | ref\|NM_000229 | gi\|4557892 |
| 33 | Control vs RA ccp neg | 58513 | epidermal growth factor receptor pathway substrate 15-like 1 | EPS15L1 | ref\|NM_021235 | gi\|10864047 |
| 34 | Control vs RA ccp neg | 1513 | cathepsin K | CTSK | ref\|NM_000396 | gi\|4503151 |
| 35 | Control vs RA ccp neg | 64434 | nucleolar protein with MIF4G domain 1 | NOM1 | ref\|NM_138400 | gi\|61097912 |
| 36 | Control vs RA ccp neg | 203068 | tubulin, beta | TUBB | ref\|NM_178014 | gi\|29788785 |
| 37 | Control vs RA ccp neg | 119504 | anaphase promoting complex subunit 16 | ANAPC16 | ref\|NM_173473 | gi\|27735039 |
| 38 | Control vs RA ccp neg | 59277 | netrin 4 | NTN4 | ref\|NM_021229 | gi\|93204871 |
| 39 | Control vs RA ccp neg | 6232 | ribosomal protein S27 | RPS27 | ref\|NM_001030 | gi\|4506711 |
| 40 | Control vs RA ccp neg | 23151 | GRAM domain containing 4 | GRAMD4 | ref\|NM_015124 | gi\|67763814 |
| 41 | Control vs RA ccp neg | 6189 | ribosomal protein S3A | RPS3A | ref\|NM_001006 | gi\|4506723 |
| 42 | Control vs RA ccp neg | 6432 | serine/arginine-rich splicing factor 7 | SRSF7 | ref\|NM_001031684 | gi\|72534660 |
| 43 | Control vs RA ccp neg | 7874 | ubiquitin specific peptidase 7 (herpes virus-associated) | USP7 | ref\|NM_003470 | gi\|150378533 |
| 44 | Control vs RA ccp neg | 6418 | SET nuclear oncogene | SET | ref\|NM_003011 | gi\|170763498 |
| 45 | Control vs RA ccp neg | 11258 | dynactin 3 (p22) | DCTN3 | ref\|NM_007234 | gi\|6005745 |
| 46 | Control vs RA ccp neg | 9733 | squamous cell carcinoma antigen recognized by T cells 3 | SART3 | ref\|NM_014706 | gi\|7661952 |
| 47 | Control vs RA ccp neg | 5217 | profilin 2 | PFN2 | ref\|NM_053024 | gi\|16753215 |
| 48 | Control vs RA ccp neg | 302 | annexin A2 | ANXA2 | ref\|NM_004039 | gi\|4757756 |
| 49 | Control vs RA ccp neg | 55830 | glycosyltransferase 8 domain containing 1 | GLT8D1 | ref\|NM_018446 | gi\|8923855 |
| 50 | Control vs RA ccp neg | 3557 | interleukin 1 receptor antagonist | IL1RN | ref\|NM_000577 | gi\|10835147 |
| 51 | Control vs RA ccp neg | 90809 | transmembrane protein 55B | TMEM55B | ref\|NM_144568 | gi\|154816184 |
| 52 | Control vs RA ccp neg | 163033 | zinc finger protein 579 | ZNF579 | ref\|NM_152600 | gi\|110681708 |
| 53 | Control vs RA ccp neg | 9605 | chromosome 16 open reading frame 7 | C16orf7 | ref\|NM_004913 | gi\|108860690 |
| 54 | Control vs RA ccp neg | 6710 | spectrin, beta, erythrocytic | SPTB | ref\|NM_001024858 | gi\|67782321 |
| 55 | Control vs RA ccp neg | 2934 | gelsolin | GSN | ref\|NM_198252 | gi\|38044288 |
| 56 | Control vs RA ccp neg | 4591 | tripartite motif containing 37 | TRIM37 | ref\|NM_015294 | gi\|15147333 |
| 57 | Control vs RA ccp neg | 6903 | tubulin folding cofactor C | TBCC | ref\|NM_003192 | gi\|4507373 |
| 58 | Control vs RA ccp neg | 11140 | cell division cycle 37 homolog (S. cerevisiae) | CDC37 | ref\|NM_007065 | gi\|5901922 |
| 59 | Control vs RA ccp neg | 7458 | eukaryotic translation initiation factor 4H | EIF4H | ref\|NM_031992 | gi\|14702180 |
| 60 | Control vs RA ccp neg | 6687 | spastic paraplegia 7 (pure and complicated autosomal recessive) | SPG7 | ref\|NM_003119 | gi\|4507173 |
| 61 | Control vs RA ccp neg | 22902 | RUN and FYVE domain containing 3 | RUFY3 | ref\|NM_014961 | gi\|7662352 |
| 62 | Control vs RA ccp neg | 6144 | ribosomal protein L21 | RPL21 | ref\|NM_000982 | gi\|18104948 |
| 63 | Control vs RA ccp neg | 84893 | F-box protein, helicase, 18 | FBX018 | ref\|NM_178150 | gi\|30795119 |
| 64 | Control vs RA ccp neg | 10295 | branched chain ketoacid dehydrogenase kinase | BCKDK | ref\|NM_005881 | gi\|171906589 |
| 65 | Control vs RA ccp neg | 3980 | ligase III, DNA, ATP-dependent | LIG3 | ref\|NM_002311 | gi\|73747844 |
| 66 | Control vs RA ccp neg | 3913 | laminin, beta 2 (laminin S) | LAM B2 | ref\|NM_002292 | gi\|119703755 |
| 67 | Control vs RA ccp neg | 221061 | family with sequence similarity 171, member A1 | FAM171A1 | ref\|NM_001010924 | gi\|63025206 |
| 68 | Control vs RA ccp neg | 790 | carbamoyl-phosphate synthetase 2, aspartate transcarbamylase, and dihydroorotase | CAD | ref\|NM_004341 | gi\|18105007 |
| 69 | Control vs RA ccp neg | 8891 | eukaryotic translation initiation factor 2B, subunit 3 gamma, 58kDa | EIF2B3 | ref\|NM_020365 | gi\|9966779 |
| 70 | Control vs RA ccp neg | 23367 | La ribonucleoprotein domain family, member 1 | LARP1 | ref\|NM_015315 | gi\|39725634 |
| 71 | Control vs RA ccp neg | 3913 | laminin, beta 2 (laminin S) | LAM B2 | ref\|NM_002292 | gi\|119703755 |
| 72 | Control vs RA ccp neg | 4000 | lamin A/C | LMNA | ref\|NM_170707 | gi\|27436946 |
| 73 | Control vs RA ccp neg | 9026 | huntingtin interacting protein 1 related | HIP1R | ref\|NM_003959 | gi\|48762942 |
| 74 | Control vs RA ccp neg | 23608 | makorin ring finger protein 1 | MKRN1 | | gi\|119604358 |
| 75 | Control vs RA ccp neg | 51585 | PCF11, cleavage and polyadenylation factor subunit, homolog (S. cerevisiae) | PCF11 | ref\|NM_015885 | gi\|33620745 |
| 76 | Control vs RA ccp neg | 10808 | heat shock 105kDa/110kDa protein 1 | HSPH1 | ref\|NM_006644 | gi\|42544159 |
| 77 | Control vs RA ccp neg | 5716 | proteasome (prosome, macropain) 26S subunit, non-ATPase, 10 | PSMD10 | ref\|NM_002814 | gi\|4506217 |
| 78 | Control vs RA ccp neg | 57662 | calmodulin regulated spectrin-associated protein family, member 3 | CAMSAP3 | ref\|NM_020902 | gi\|130502140 |

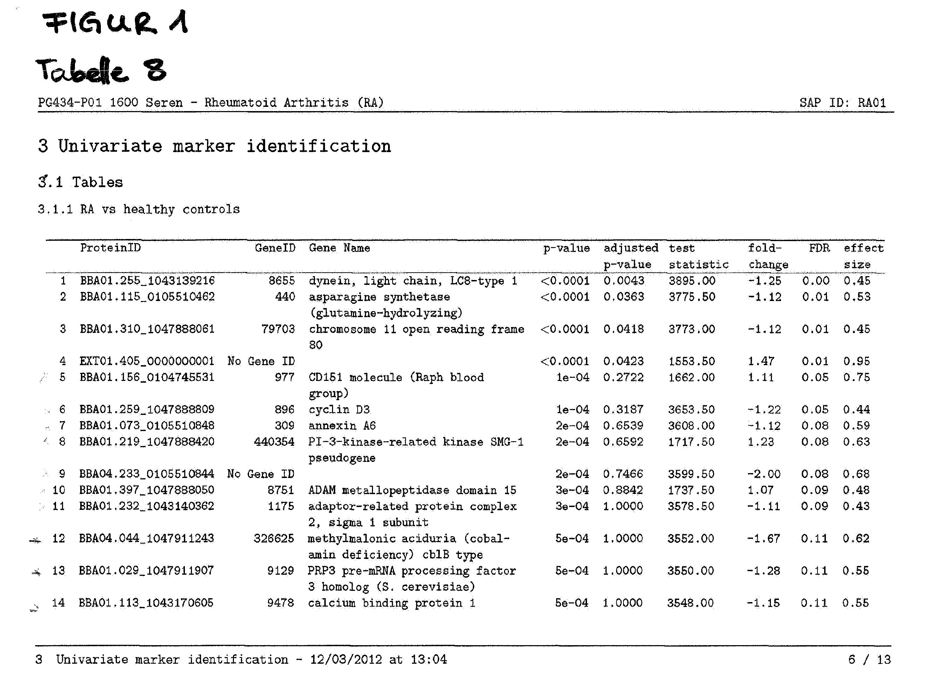

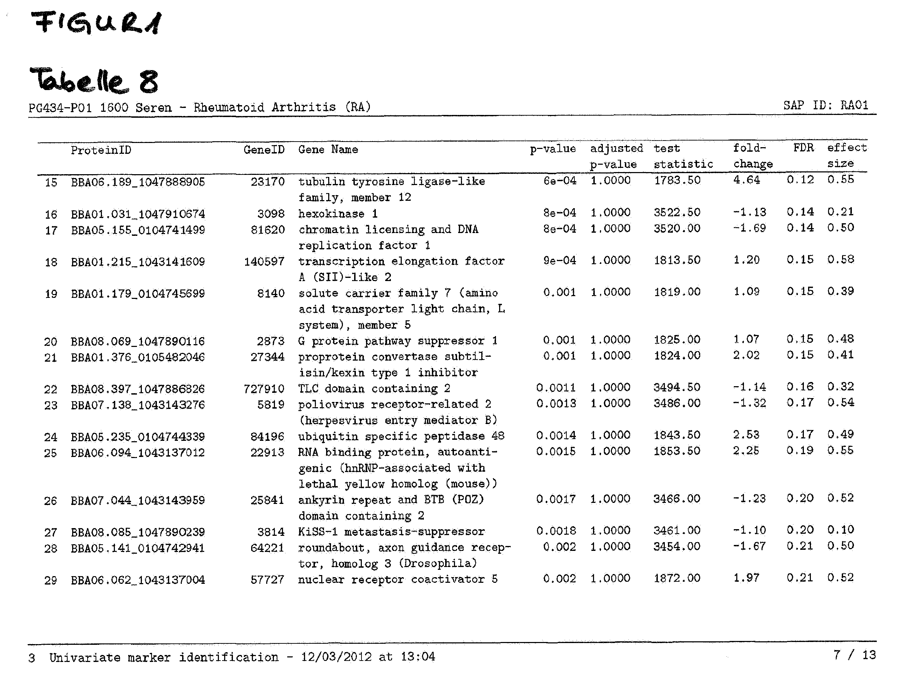

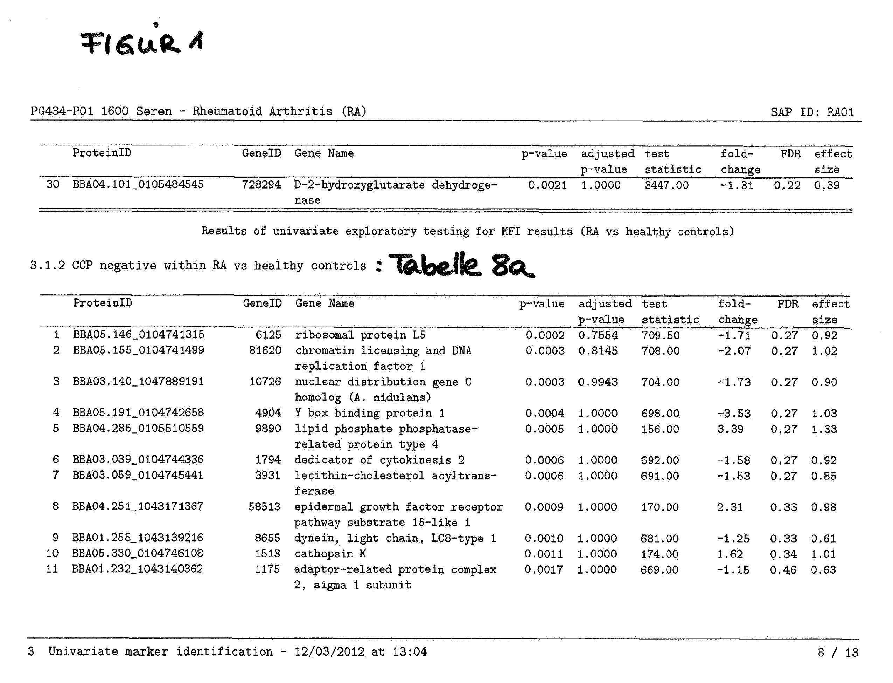

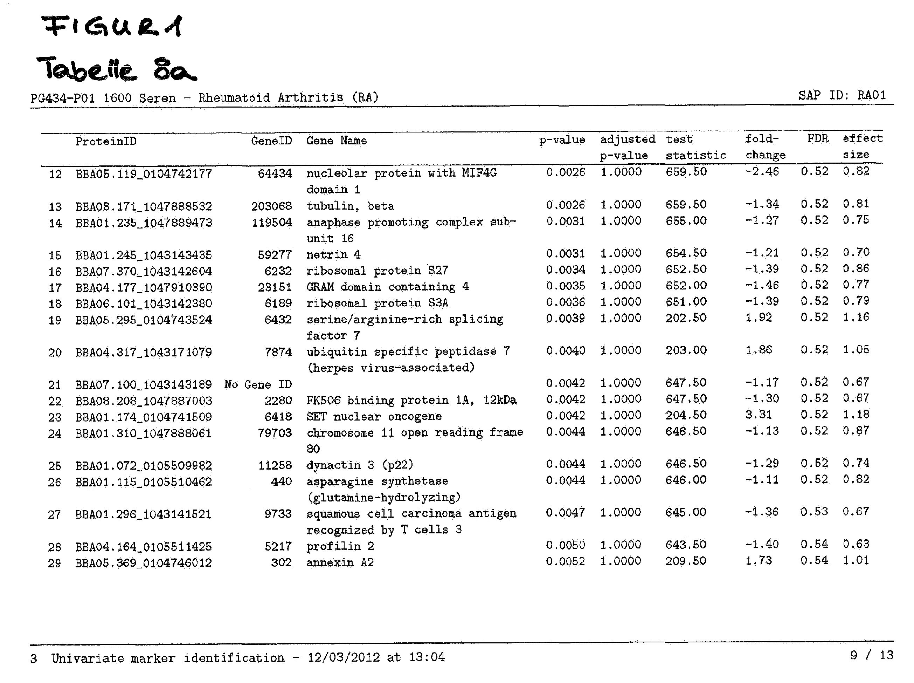

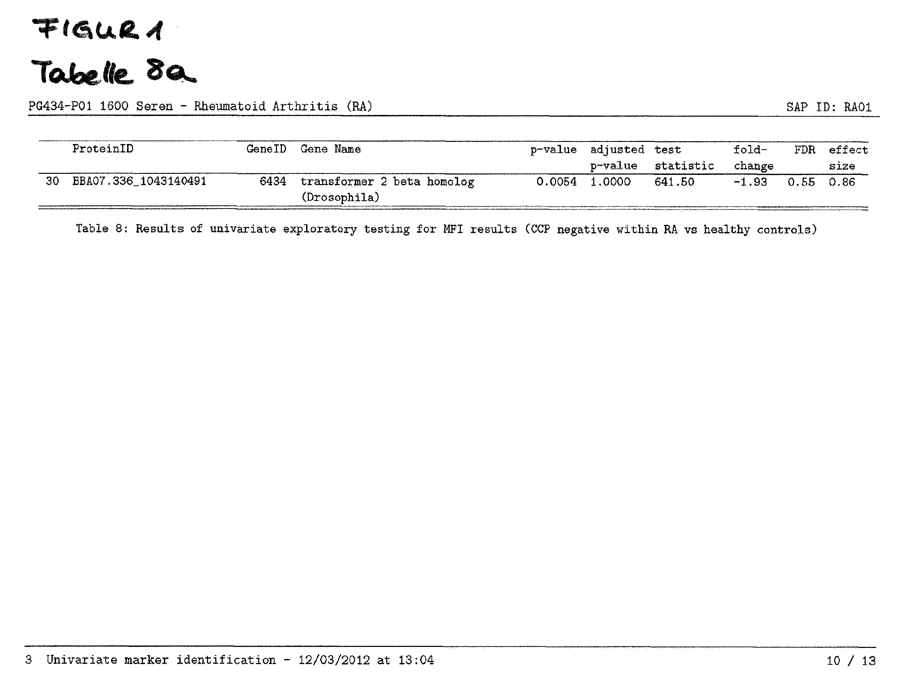

Die Statistischen Ergebnisse zu den validierten Markersequenzkandidaten (erfindungsgemäße Markersequenzen für Rheumatoide Arthritis) sind in den Tabellen 8 und 8a (Figur 1) angegeben.

## Patentansprüche

1. Verwendung von Markersequenzen zur Diagnose von Rheumatoider Arthritis, wobei mindestens eine Markersequenz ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfasst und / oder ein durch die Sequenzen 1 bis 156 kodiertes Protein oder eine Teilsequenz oder Fragment davon an oder von einem zu untersuchenden Patienten bestimmt wird und wobei die Markersequenz(en) mit einem Verfahren identifiziert wurde(n), umfassend die
e) Identifizierung von Markersequenzkandidaten durch differentielles Screening mit Proteinbiochips aus jeweils einer cDNA-Expressionsbank eines Patienten mit Rheumatoider Arthritis und einem Probanden ohne Rheumatoide Arthritis,
f) Expression der Markersequenzkandidaten zur Herstellung der kodierten Proteine und / oder Teilproteine (Peptide),
g) Herstellung von Luminex-Beads, an die ein oder mehrere der in Schritt c) hergestellten Proteine und / oder Teilproteine (Peptide) gekoppelt sind wobei an die Luminex-Beads gegebenenfalls auch bereits bekannte Biomarker für Rheumatoide Arthritis, beispielsweise CCP, gekoppelt sind,
h) Validierung der Markersequenzkandidaten mittels Proben von Patienten mit Rheumatoider Arthritis und Probanden ohne Rheumatoide Arthritis, und wobei die Validierung gegebenenfalls in Gegenwart der bereits bekannten Biomarker für Rheumatoide Arthritis, beispielsweise CCP, durchgeführt wird.

2. Verwendung der Markersequenzen zur Diagnose von Rheumatoider Arthritis nach Anspruch 1, wobei mindestens eine Markersequenz ausgewählt aus der Gruppe der Teilsequenzen SEQ ID No. 29 bis 78 und / oder cDNAs SEQ ID No. 108 bis 156 und / oder der genomischen Sequenzen, die eine der Sequenzen SEQ ID No. 29 bis 78 oder 108 bis 156 umfasst und / oder ein durch die Sequenzen 29 bis 78 oder 108 bis 156 kodiertes Protein oder eine Teilsequenz oder Fragment davon an oder von einem zu untersuchenden Patienten bestimmt wird und wobei die Markersequenz(en) mit einem Verfahren identifiziert wurde(n), umfassend die Schritte a) bis d) und
wobei die Schritte c) und d) in Gegenwart von CPP durchgeführt wurden und
wobei die Markersequenzen sensitiver im Bezug auf die Diagnose von Rheumatoider Arthritis sind als CCP.

3. Verwendung der Markersequenzen zur Diagnose von Rheumatoider Arthritis nach einem der Ansprüche 1 oder 2, **dadurch gekennzeichnet, dass** 2 oder 3, vorzugsweise 4 oder 5, besonders bevorzugt 6, 7 oder 8 oder mehr verschiedene Markersequenzen, beispielsweise 10 bis 20 oder 30 oder mehr verschiedene Markersequenzen an oder von einem zu untersuchenden Patienten bestimmt werden.

4. Verwendung der Markersequenz(en)zur Diagnose von Rheumatoider Arthritis nach einem der vorgehenden Ansprüche, **dadurch gekennzeichnet, dass** die Bestimmung mittels in-vitro Diagnose erfolgt.

5. Verwendung mindestens einer Markersequenz ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfasst und / oder eines durch die Sequenzen 1 bis 156 kodierten Proteins oder einer Teilsequenz oder eines Fragments davon als Diagnostikum, wobei die Markersequenz(en) mit einem Verfahren identifiziert wurde(n), umfassend die Schritte
e) Identifizierung von Markersequenzkandidaten durch differentielles Screening mit Proteinbiochips aus jeweils einer cDNA-Expressionsbank eines Patienten mit Rheumatoider Arthritis und einem Probanden ohne Rheumatoide Arthritis,
f) Expression der Markersequenzkandidaten zur Herstellung der kodierten Proteine und / oder Teilproteine (Peptide),
g) Herstellung von Luminex-Beads, an die ein oder mehrere der in Schritt c) hergestellten Proteine und / oder Teilproteine (Peptide) gekoppelt sind wobei an die Luminex-Beads gegebenenfalls auch bereits bekannte Biomarker für Rheumatoide Arthritis, beispielsweise CCP, gekoppelt sind,
h) Validierung der Markersequenzkandidaten mittels Proben von Patienten mit Rheumatoider Arthritis und Probanden ohne Rheumatoide Arthritis, und wobei die Validierung gegebenenfalls in Gegenwart der bereits bekannten Biomarker für Rheumatoide Arthritis, beispielsweise CCP, durchgeführt wird.

6. Verwendung der Markersequenz(en) zur Diagnose von Rheumatoider Arthritis nach einem der vorhergehenden Ansprüche, **dadurch gekennzeichnet, dass** die Markersequenz(en) auf einem festen Träger aufgebracht wird (werden), wobei der feste Träger beispielsweise ausgewählt wird aus den festen Trägern umfassend Filter, Membranen, Wafer, beispielsweise Silizium-Wafer, Glas, Metall, Kunststoff, Chips, massenspektrometrische Targets, Matrix, Kügelchen (Beads), beispielsweise magnetische, beschichtete oder markierte Kügelchen (Beads), wie Fluorophor-markierte Kügelchen oder Luminex-Beads.

7. Verfahren zur Diagnose von Rheumatoider Arthritis, wobei
a.) mindestens eine Markersequenz ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfasst und / oder eines durch die Sequenzen 1 bis 156 kodierten Proteins oder einer Teilsequenz oder eines Fragments davon auf einem festen Träger, vorzugsweise auf einem Kügelchen (Bead) aufgebracht wird (werden) und
b.) mit Körperflüssigkeit oder Gewebeauszug eines Patienten in Kontakt gebracht wird (werden) und c.) der Nachweis einer Wechselwirkung der Körperflüssigkeit oder Gewebeauszug mit der (den) Markersequenz(en) aus a.) erfolgt.

8. Verfahren zum Stratifizieren, insbesondere zur Risikostratifizierung, oder zur Therapiesteuerung eines Patienten mit Rheumatoider Arthritis, wobei mindestens eine Markersequenz ausgewählt aus der Gruppe der Teilsequenzen SEQ ID No. 1 bis 78 und / oder der cDNAs SEQ ID No. 79 bis 156 und / oder den genomischen Sequenzen, die eine der Sequenzen 1 bis 156 umfassen und / oder eines durch die Sequenzen 1 bis 156 kodierten Proteins oder einer Teilsequenz oder eines Fragments davon an oder von einem zu untersuchenden Patienten bestimmt wird.

9. Verfahren nach Anspruch 8, wobei das Stratifizieren oder die Therapiesteuerung Entscheidungen zur Behandlung und Therapie des Patienten, insbesondere Hospitalisierung des Patienten, Einsatz, Wirkung und /oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst.

10. Assay, Proteinbiochip bestehend aus einer Anordnung enthaltend mindestens eine Markersequenz ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfassen und / oder eines durch die Sequenzen 1 bis 156 kodierten Proteins oder einer Teilsequenz oder eines Fragments davon.

11. Verwendung eines Assays nach Anspruch 10 zum Identifizieren und Charakterisieren einer Substanz für Rheumatoide Arthritis enthaltend Mittel zum Nachweis eines Bindungserfolges, **dadurch gekennzeichnet, dass** eine Anordnung oder Assay mit a.) mindestens einer zu untersuchenden Substanz in Kontakt gebracht wird und b.) ein Bindungserfolg nachgewiesen wird.

12. Diagnostikum zur Diagnose von Rheumatoider Arthritis enthaltend mindestens eine Markersequenz ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfasst und / oder eines durch die Sequenzen 1 bis 156 kodierten Proteins oder einer Teilsequenz oder eines Fragments davon und wobei die Markersequenz(en) mit einem Verfahren identifiziert wurde(n), umfassend die Schritte
a.)Identifizierung von Markersequenzkandidaten durch differentielles Screening mit Proteinbiochips aus jeweils einer cDNA-Expressionsbank eines Patienten mit Rheumatoider Arthritis und einem Probanden ohne Rheumatoide Arthritis,
b.)Expression der Markersequenzkandidaten zur Herstellung der kodierten Proteine und / oder Teilproteine (Peptide),
c.)Herstellung von Luminex-Beads, an die ein oder mehrere der in Schritt c) hergestellten Proteine und / oder Teilproteine (Peptide) gekoppelt sind wobei an die Luminex-Beads gegebenenfalls auch bereits bekannte Biomarker für Rheumatoide Arthritis, beispielsweise CCP, gekoppelt sind,
d.)Validierung der Markersequenzkandidaten mittels Proben von Patienten mit Rheumatoider Arthritis und Probanden ohne Rheumatoide Arthritis, und wobei die Validierung gegebenenfalls in Gegenwart der bereits bekannten Biomarker für Rheumatoide Arthritis, beispielsweise CCP, durchgeführt wird.

13. Target zur Behandlung und Therapie von Rheumaoider Arthritis ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfasst und / oder eines durch die Sequenzen 1 bis 156 kodierten Proteins oder einer Teilsequenz oder eines Fragments davon.

14. Verwendung einer oder mehrerer Markersequenz ausgewählt aus der Gruppe der Teilsequenzen SEQ ID No. 1 bis 78 und / oder der cDNAs SEQ ID No. 79 bis 156 und / oder der genomischen Sequenzen, die eine der Sequenzen 1 bis 156 umfassen und / oder eines durch die Sequenzen 1 bis 156 kodierten Proteins oder einer Teilsequenz oder eines Fragments davon als Affinitätsmaterial zur Durchführung einer Apherese oder Blutwäsche für Patienten mit Prostatakarzinom.

15. Verwendung mindestens einer Markersequenz ausgewählt aus der Gruppe der Teilsequenzen SEQ ID No. 29 bis 78 und / oder der cDNAs SEQ ID No. 108 bis 156 und / oder der genomischen Sequenzen, die eine der Sequenzen SEQ ID No. 29 bis 78 oder 108 bis 156 umfassen und / oder eines durch die Sequenzen 29 bis 78 oder 108 bis 156 kodierten Proteins oder einer Teilsequenz oder eines Fragments davon zur Identifizierung einer Subgruppe von Patienten innerhalb der Gruppe der Patienten mit Rheumatoider Arthritis, wobei die Patienten der Subgruppe nicht mittels des Markers CCR identifiziert werden können.

16. Markersequenz für Rheumatoide Arthritis ausgewählt aus der Gruppe umfassend die Teilsequenzen SEQ ID No. 1 bis 78 und die durch die Sequenzen 1 bis 78 kodierten Protein und Teilsequenzen und Fragmente davon.
